(19) Europäisches Patentamt
European Patent Office
Office européen des brevets

(11) Veröffentlichungsnummer: **0 084 357**
**A1**

# (12) EUROPÄISCHE PATENTANMELDUNG

(21) Anmeldenummer: **83100295.1**

(22) Anmeldetag: **14.01.83**

(51) Int. Cl.³: **C 07 D 243/26**
C 07 D 401/12, C 07 D 405/12
C 07 H 13/12, A 61 K 31/55
//C07C125/065

(30) Priorität: **19.01.82 CH 313/82**

(43) Veröffentlichungstag der Anmeldung: **27.07.83 Patentblatt 83/30**

(84) Benannte Vertragsstaaten: **AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Cassal, Jean-Marie, Dr.**
**4 rue du Markstein**
**F-68100 Mulhouse(FR)**

(72) Erfinder: **Fischli, Albert Eduard, Prof. Dr.**
**Im Wenkenberg 20**
**CH-4125 Riehen(CH)**

(72) Erfinder: **Szente, André, Dr.**
**Baselstrasse 70**
**CH-4125 Riehen(CH)**

(74) Vertreter: **Lederer, Franz, Dr. et al,**
**Patentanwälte Dr. Lederer Franz Meyer-Roxlau Reiner F.**
**Lucile-Grahn-Strasse 22**
**D-8000 München 80(DE)**

(54) **Benzodiazepine, ihre Herstellung und diese enthaltende Arzneimittel.**

(57) Es werden neue pharmazeutische Wirkstoffe beschrieben, welche die intestinale Resorption von Cholesterin hemmen und demnach bei der Bekämpfung bzw. Verhütung der Atherosklerose verwendet werden können. Diese Wirkstoffe, nämlich Benzodiazepine der allgemeinen Formel

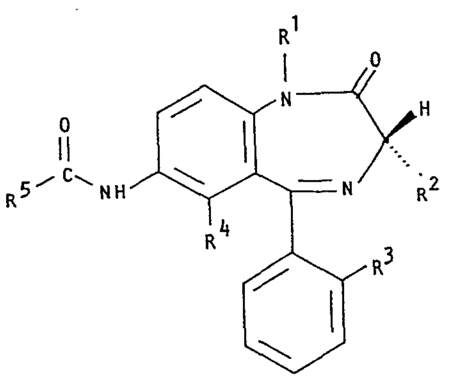

1

worin $R^1$ ($C_1$-$C_4$)-Alkyl, $R^2$ Wasserstoff oder Methyl, $R^3$ und $R^4$ je Halogen und $R^5$ durch mindestens zwei Hydroxygruppen substituiertes ($C_3$-$C_9$)-Alkylamino, durch mindestens eine Hydroxygruppe substituiertes ($C_3$-$C_6$)-Cycloalkylamino, Glucosamino, Galactosamino, Mannosamino, Monohydroxy-1-azetidinyl, Mono- oder Dihydroxy-1-pyrrolidinyl oder Mono-, Di- oder Trihydroxy-1-piperidinyl bedeuten,

leicht hydrolysierbare Ester und Aether davon, sowie pharmazeutisch annehmbare Salze von diesen Verbindungen, weisen nur eine geringe Toxizität auf und können in Form von pharmazeutischen Präparaten als Heilmittel verwendet werden.

EP 0 084 357 A1

Croydon Printing Company Ltd.

F.Hoffmann-La Roche & Co. Aktiengesellschaft, 0084357

RAN 4008/328

Benzodiazepine,

ihre Herstellung und diese enthaltende Arzneimittel.

Die vorliegende Erfindung betrifft Benzodiazepine. Im speziellen betrifft sie Benzodiazepine der allgemeinen Formel

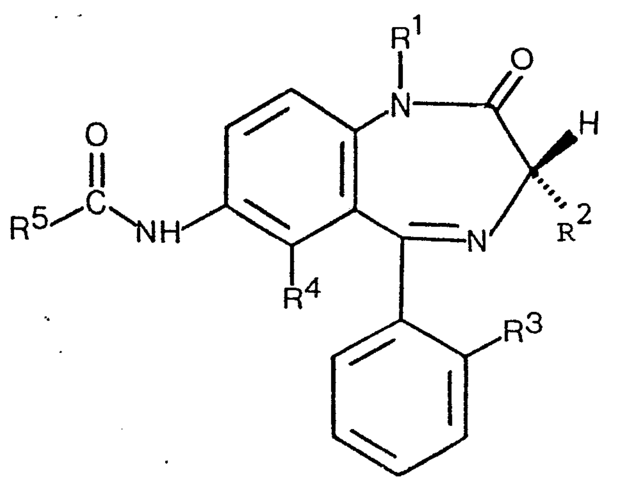

I

worin $R^1$ $(C_1-C_4)$-Alkyl, $R^2$ Wasserstoff oder Methyl, $R^3$ und $R^4$ je Halogen und $R^5$ durch mindestens zwei Hydroxygruppen substituiertes $(C_3-C_9)$-Alkylamino, durch mindestens eine Hydroxygruppe substituiertes $(C_3-C_6)$-Cycloalkylamino, Glucosamino, Galactosamino, Mannosamino, Monohydroxy-1-azetidinyl, Mono- oder Dihydroxy-1-

Nt/ 4.11.82

pyrrolidinyl oder Mono-, Di- oder Trihydroxy-1-piperidinyl, bedeuten,

leicht hydrolysierbare Ester und Aether davon, sowie pharmazeutisch annehmbare Salze von diesen Verbindungen.

Diese Produkte sind neu; sie zeichnen sich durch wertvolle pharmakologische Eigenschaften aus und können bei der Bekämpfung bzw. Verhütung von Krankheiten verwendet werden.

Gegenstand der vorliegenden Erfindung sind Benzodiazepine der obigen Formel I, ihre leicht hydrolysierbaren Ester und Aether, sowie pharmazeutisch annehmbare Salze von diesen Verbindungen als solche und als pharmazeutische Wirkstoffe, die Herstellung dieser Produkte, ferner Arzneimittel, enthaltend ein solches Produkt, und die Herstellung solcher Arzneimittel.

Der Ausdruck "$(C_1-C_4)$-Alkyl" bezeichnet geradkettige oder verzweigte, gesättigte Kohlenwasserstoffreste mit 1-4 Kohlenstoffatomen, wie Methyl, Aethyl, n-Propyl, Isopropyl, n-Butyl und dergleichen. Der Ausdruck "durch mindestens zwei Hydroxygruppen substituiertes $(C_3-C_9)$-Alkylamino" bezeichnet geradkettige oder verzweigte, gesättigte Kohlenwasserstoffreste, welche einerseits über die Gruppe -NH- verknüpft und andererseits durch mindestens zwei, jedoch höchstens vier Hydroxygruppen substituiert sind, wobei die Hydroxygruppen sich nicht in α-Stellung zur Gruppe -NH- befinden können. Vorzugsweise umfasst dieser Ausdruck Reste mit 3-6 Kohlenstoffatomen und zwei oder drei Hydroxygruppen, wie 1,1-bis(Hydroxymethyl)äthylamino, 2-Hydroxy-1,1-bis(hydroxymethyl)äthylamino, 2-Hydroxy-1-(hydroxymethyl)äthylamino, 2,3-Dihydroxypropylamino und dergleichen.

Der Ausdruck "durch mindestens eine Hydroxygruppe substituiertes $(C_3-C_6)$-Cycloalkylamino" bezeichnet gesättigte cyclische Kohlenwasserstoffreste, welche einerseits über die Gruppe -NH- verknüpft und andererseits durch mindestens eine, jedoch höchstens drei Hydroxygruppen substituiert sind, wobei die Hydroxygruppe(n) sich nicht in α-Stellung zur Gruppe -NH- befinden kann (können). Die maximale Anzahl der Hydroxygruppen richtet sich nach der Anzahl Kohlenstoffatome. Als Beispiel sei 4-Hydroxycyclohexylamino genannt.

Auch die Hydroxygruppen in den Substituenten Monohydroxy-1-azetidinyl, Mono- oder Dihydroxy-1-pyrrolidinyl oder Mono-, Di- oder Trihydroxy-1-piperidinyl können sich nicht in α-Stellung zum Stickstoffatom befinden.

Der Ausdruck "Halogen" bedeutet Fluor, Chlor, Brom oder Jod.

Der Ausdruck "leicht hydrolysierbare Ester und Aether davon" bedeutet, dass es sich um Derivate von Benzodiazepinen der Formel I handelt, bei welchen die im Rest $R^5$ vorhandene(n) Hydroxygruppe(n) allesamt oder teilweise in Form leicht hydrolysierbarer Ester und/oder Aether vorliegen können. Derivate von Benzodiazepinen der obigen Formel I, welche auf diese Weise abgewandelte Hydroxygruppen enthalten, sind zum Teil selber pharmakologisch wirksam oder stellen biologische Vorläufer von pharmakologisch wirksamen Verbindungen dar. Der Ausdruck "leicht hydrolysierbar" soll somit Ester und Aether bezeichnen, welche unter physiologischen Bedingungen leicht gespalten werden. Leicht hydrolysierbare Ester sind zum Beispiel Ester bzw. Halbester mit niederen Alkancarbonsäuren bzw. niederen Alkandicarbonsäuren (d.h. beispielsweise Acetate, Formiate, Propionate, Hemisuccinate und dergleichen), cyclische Diester mit niederen Alkandicarbonsäuren (sofern mindestens zwei freie Hydroxygruppen vorhanden sind), aromatische Carbonsäureester, z.B. Benzoate, araliphatische Carbonsäureester, z.B. Phenylessigsäureester, und dergleichen. Leicht hydrolysierbare Aether sind zum Beispiel offenkettige oder cyclische Acetale oder Ketale, beispielsweise Tetrahydropyranyläther,

Methoxymethyläther, 1,3-Dioxolane (sofern mindestens zwei freie Hydroxygruppen vorhanden sind), niedere Alkyläther oder dergleichen. Als Beispiele für derart abgewandelte Reste $R^5$ können die folgenden genannt werden: 2-(Acetyloxy)-1-[(acetyloxy)methyl]-1-methyläthylamino, 2-(Acetyloxy)-1-(hydroxymethyl)-1-methyläthylamino, (2,2-dimethyl-1,3-dioxolan-4-yl)methylamino, 4-Acetyloxy-1-piperidinyl und dergleichen. Die vorliegende Erfindung umfasst auch andere pharmazeutisch annehmbare biologische Vorläufer von Benzodiazepinen der obigen Formel I, welche nach pharmazeutischer Verabreichung unter physiologischen Bedingungen in die Verbindungen der obigen Formel I übergeführt werden.

Der Ausdruck "nieder" bedeutet, dass die derart bezeichneten Verbindungen oder Reste bis zu 7 Kohlenstoffatome, vorzugsweise bis zu 4 Kohlenstoffatome enthalten und geradkettig oder verzweigt sein können.

Unter den erfindungsgemässen Produkten sind diejenigen bevorzugt, worin $R^1$ Methyl bedeutet. Die bevorzugte Bedeutungsmöglichkeit von $R^2$ ist Methyl. $R^3$ bedeutet vorzugsweise Chlor. $R^4$ bedeutet vorzugsweise Brom. Vorzugsweise handelt es sich um Benzodiazepine der Formel I oder pharmazeutisch annehmbare Säureadditionssalze davon, und hierin bedeutet $R^5$ vorzugsweise durch mindestens zwei Hydroxygruppen substituiertes $(C_3-C_9)$-Alkylamino.

Eine im Rahmen der vorliegenden Erfindung ganz besonders bevorzugte Verbindung ist 1-[(S)-6-Brom-5-(o-chlorphenyl)-2,3-dihydro-1,3-dimethyl-2-oxo-1H-1,4-benzodiazepin-7-yl]-3-[1,1-bis(hydroxymethyl)äthyl]harnstoff.

Weitere bevorzugte Verbindungen der Formel I sind:

1-[(S)-6-Brom-5-(o-chlorphenyl)-2,3-dihydro-1,3-dimethyl-2-oxo-1H-1,4-benzodiazepin-7-yl]-3-[2-hydroxy-1,1-bis(hydroxymethyl)äthyl]harnstoff,

1-[(S)-6-Brom-5-(o-chlorphenyl)-2,3-dihydro-1,3-dimethyl-2-oxo-1H-1,4-benzodiazepin-7-yl]-3-[2-hydroxy-1-(hydroxymethyl)äthyl]harnstoff,

1-[(S)-6-Brom-5-(o-chlorphenyl)-2,3-dihydro-1,3-dimethyl-2-oxo-1H-1,4-benzodiazepin-7-yl]-3-(2,3-dihydroxypropyl)harnstoff,

1-[(S)-6-Brom-5-(o-chlorphenyl)-2,3-dihydro-1,3-dimethyl-2-oxo-1H-1,4-benzodiazepin-7-yl]-3-(4-hydroxycyclohexyl)harnstoff,

N-[(S)-6-Brom-5-(o-chlorphenyl)-2,3-dihydro-1,3-dimethyl-2-oxo-1H-1,4-benzodiazepin-7-yl]-4-hydroxy-1-piperidincarboxamid,

N-[(S)-6-Brom-5-(o-chlorphenyl)-2,3-dihydro-1,3-dimethyl-2-oxo-1H-1,4-benzodiazepin-7-yl]-3-hydroxy-1-piperidincarboxamid und

2-[3-[(S)-6-Brom-5-(o-chlorphenyl)-2,3-dihydro-1,3-dimethyl-2-oxo-1H-1,4-benzodiazepin-7-yl]ureido]-2-deoxy-D-glucose.

Die Benzodiazepine der eingangs definierten Formel I, ihre leicht hydrolysierbaren Ester und Aether, sowie die pharmazeutisch annehmbaren Salze davon können erfindungsgemäss dadurch hergestellt werden, dass man

a) ein Benzodiazepin der allgemeinen Formel

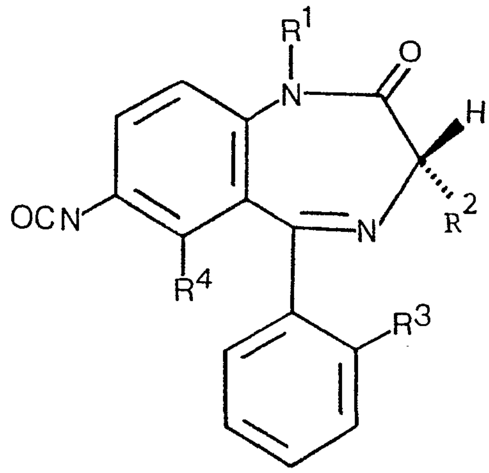

II

worin $R^1$, $R^2$, $R^3$ und $R^4$ obige Bedeutung besitzen, mit einer Verbindung der allgemeinen Formel

$$R^5\text{-}H \qquad III$$

worin $R^5$ obige Bedeutung besitzt, oder mit einem Derivat einer Verbindung der Formel III, worin die Hydroxygruppe(n) allesamt oder teilweise in Form leicht hydrolysierbarer Ester und/oder Aether vorliegt bzw. vorliegen, umsetzt,

b) erwünschtenfalls einen erhaltenen leicht hydrolysierbaren Ester oder Aether eines Benzodiazepins der Formel I hydrolysiert, und/oder

c) erwünschtenfalls in einer erhaltenen Verbindung die freie(n) Hydroxygruppe(n) allesamt oder teilweise in leicht hydrolysierbare Ester- oder Aethergruppen überführt und/oder

d) erwünschtenfalls ein erhaltenes Benzodiazepin der Formel I oder einen leicht hydrolysierbaren Ester oder Aether davon in ein pharmazeutisch annehmbares Salz überführt.

Für die Herstellung der erfindungsgemässen Produkte nach Verfahrensvariante a) geht man zweckmässigerweise so vor, dass man das zum Einsatz vorgesehene Benzodiazepin der Formel II kurz oder unmittelbar vor der Umsetzung mit der Aminoverbindung der Formel III oder einem leicht hydrolysierbaren Ester und/oder Aether davon auf die weiter unten beschriebene Weise aus einem entsprechenden Benzodiazepin der allgemeinen Formel IV herstellt und das betreffende Benzodiazepin der Formel II als Rohprodukt in die Reaktion einbringt.

Zu einer Lösung des Benzodiazepins der Formel II in einem der weiter unten erwähnten inerten organischen Lösungsmittel kann man dann eine Aminoverbindung der Formel III oder einen leicht hydrolysierbaren Ester und/oder Aether davon geben. Hierbei kann die Aminoverbindung in Form einer

Lösung oder auch ohne Lösungsmittel verwendet werden.

Man kann aber ohne weiteres auch umgekehrt verfahren, d.h. die das Benzodiazepin der Formel II enthaltende Lösung zur Aminoverbindung geben, wobei sich die Aminoverbindung zweckmässigerweise in Lösung befindet.

Als Lösungsmittel für das Benzodiazepin der Formel II eignen sich beispielsweise halogenierte Kohlenwasserstoffe, wie 1,2-Dichloräthan, Methylenchlorid, Chloroform, o-Dichlorbenzol und dergleichen, Aether, wie Tetrahydrofuran, Dioxan, Dimethoxyäthan, Diäthylenglykoldimethyläther und dergleichen, Aceton und dergleichen. Als Lösungsmittel für das Amin der Formel III oder einen leicht hydrolysierbaren Ester und/oder Aether davon eignen sich die vorstehend erwähnten, gegebenenfalls Mischungen davon mit Wasser oder Wasser.

Die Reaktion einer Verbindung der Formel II mit einer Aminoverbindung der Formel III oder einem leicht hydrolysierbaren Ester und/oder Aether davon erfolgt zweckmässigerweise bei Raumtemperatur oder Temperaturen unterhalb davon.

Gemäss Verfahrensvariante b) können leicht hydrolysierbare Ester und/oder Aether von Benzodiazepinen der Formel I hydrolysiert werden. Die Hydrolyse von entsprechenden Estern erfolgt dabei vorzugsweise unter milden alkalischen Bedingungen oder enzymatisch mit einer Esterase, währenddem entsprechende Aether vorzugsweise unter milden sauren Bedingungen gespalten werden. Es handelt sich dabei durchwegs um dem Fachmann bekannten Methoden. Es gilt noch zu beachten, dass natürlich nur solche Methoden verwendet werden können, welche andere im Molekül vorhandene Strukturelemente nicht in Mitleidenschaft ziehen.

Gemäss Verfahrensvariante c) können freie Hydroxygruppen in leicht hydrolysierbare Ester- und/oder Aethergruppen übergeführt werden. Die Herstellung von leicht hydrolysierbaren Estern und/oder Aethern erfolgt dabei durchwegs nach an sich bekannten und jedem Fachmann geläufigen Methoden. Leicht hydrolysierbare Ester können beispielsweise so hergestellt werden, dass man eine entsprechende Hydroxyverbindung mit einem entsprechenden Carbonsäurechlorid, einem entsprechenden Carbonsäureanhydrid oder mit einem anderen reaktionsfähigen Carbonsäurederivat umsetzt. Als Lösungsmittel eignen sich dabei inerte organische Lösungsmittel, z.B. halogenierte Kohlenwasserstoffe, wie Methylenchlorid, Chloroform, 1,2-Dichloräthan und dergleichen, Aether, wie Tetrahydrofuran, Diäthyläther und dergleichen, Aceton, Dimethylformamid, Dimethylsulfoxid und dergleichen.

Leicht hydrolysierbare Aether können beispielsweise so hergestellt werden, dass man eine entsprechende Hydroxyverbindung mit einem Halogenid, wie Chlordimethyläther oder dergleichen, umsetzt, und zwar in Gegenwart einer Base und in einem der oben erwähnten inerten organischen Lösungsmittel, oder dass man eine entsprechende Hydroxyverbindung in Gegenwart einer Säure mit einem Enoläther, wie 3,4-Dihydropyran, umsetzt, oder dass man eine entsprechende Hydroxyverbindung, welche mindestens zwei Hydroxygruppen besitzt, in Gegenwart einer Säure mit einem Aldehyd oder einem Keton, wie Aceton, umsetzt.

Gemäss Verfahrensvariante d) können die Benzodiazepine der obigen Formel I und ihre leicht hydrolysierbaren Ester und Aether in pharmazeutisch annehmbare Salze übergeführt werden. Die Herstellung von solchen pharmazeutisch annehmbaren Salzen erfolgt dabei nach allgemein üblichen Methoden. Als Säureadditionssalze kommen sowohl Salze mit pharmazeutisch annehmbaren, anorganischen als auch Salze mit pharmazeutisch annehmbaren organischen Säuren in Betracht, beispielsweise Hydrochloride, Hydrobromide, Sulfate, Zitrate, Acetate, Succinate, Methansulfonate, p-Toluolsulfonate und dergleichen. Liegt eine der im Rest $R^5$ vorhan-

denen Hydroxygruppen in Form eines Halbesters mit einer niederen Alkandicarbonsäure vor, so kommen auch Salze mit pharmazeutisch annehmbaren Basen in Betracht, beispielsweise Natrium-, Kalium- oder Kalziumsalze, oder Salze mit organischen Basen, z.B. mit Piperidin, Diäthylamin, N-methylglucamin, Triäthylamin, N-(2-aminoäthyl)glycin oder dergleichen.

Die als Ausgangsstoffe verwendeten Benzodiazepine der Formel II können - wie bereits weiter oben erwähnt - aus entsprechenden Benzodiazepinen der allgemeinen Formel

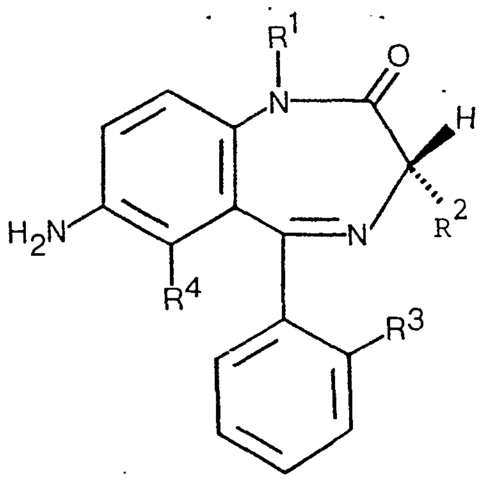

IV

worin $R^1$, $R^2$, $R^3$ und $R^4$ obige Bedeutung besitzen, hergestellt werden, und zwar durch Umsetzen mit Phosgen. Dabei geht man zweckmässigerweise so vor, dass man eine Lösung von Phosgen in einem unter den Reaktionsbedingungen inerten organischen Lösungsmittel vorlegt und dann unter Kühlen eine Lösung eines Benzodiazepines der Formel IV zugibt, hierauf einige Zeit unter Rückfluss zum Sieden erhitzt, dann wieder abkühlt und schliesslich die erhaltene Lösung mit einer tertiären organischen Aminoverbindung, wie Triäthylamin, basisch oder mindestens neutral stellt. Die erhaltene Lösung, enthaltend ein Benzodiazepin der Formel II, kann unter Feuchtigkeitsausschluss und in der Kälte während mehreren Stunden aufbewahrt werden. Durch Eindampfen derselben erhält man das rohe Benzodiazepin der Formel II, das unter Feuchtigkeitsausschluss und in der Kälte während mehreren Tagen aufbewahrt werden kann. Zweckmässigerweise wird das Benzodiazepin der Formel II nicht gereinigt, sondern in roher Form weiterverarbeitet.

Die Verbindungen der Formel IV gehören einer an sich bekannten Verbindungsklasse an, und es sind bereits diverse spezifische Vertreter dieser Verbindungsklasse in der Literatur beschrieben worden. Noch nicht spezifisch vorbeschriebene Vertreter dieser Verbindungsklasse können nach an sich bekannten und jedem Fachmann geläufigen Methoden hergestellt werden. Detaillierte Angaben betreffend die Herstellung von Verbindungen der Formel IV können auch den nachfolgenden Beispielen entnommen werden.

Die Benzodiazepine der Formel I und zum Teil auch ihre leicht hydrolysierbaren Ester und Aether hemmen die intestinale Resorption von Cholesterin.

Die Hemmung der intestinalen Resorption von Cholesterin lässt sich tierexperimentell wie folgt nachweisen:

Die zu untersuchenden Substanzen werden Gruppen von je 6 normalen weiblichen Albino-Ratten (Körpergewicht etwa 70 g) mittels einer Magensonde verabreicht. Unmittelbar anschliessend erhalten die Tiere mittels einer Magensonde eine radioaktives Cholesterin enthaltende Testnahrung. Hierauf wird der Kot während drei Tagen gesammelt, gefriergetrocknet und pulverisiert, und ein Aliquot wird zwecks Ermittlung der Radioaktivität des Kots verbrannt. Unbehandelte Kontrolltiere scheiden etwa 40-50% des mit der Nahrung aufgenommenen radioaktiven Cholesterins aus. Das nicht ausgeschiedene radioaktive Cholesterin wird als resorbiertes Cholesterin angesehen, wobei die Menge an resorbiertem Cholesterin bei unbehandelten Kontrolltieren arbiträr als 100% festgesetzt wird. Tiere, welche mit einer die intestinale Resorption von Cholesterin hemmenden Substanz behandelt worden sind, zeigen eine geringere Resorption von radioaktivem Cholesterin als unbehandelte Kontrolltiere. Die Cholesterinresorption (CHORES) wird in Prozenten

der Cholesterinresorption von unbehandelten Kontrolltieren ausgedrückt.

In der nachfolgenden Tabelle werden die Resultate dargestellt, welche mit einigen repräsentativen erfindungsgemässen Produkten erhalten worden sind. Angegeben werden für jede der darin figurierenden Verbindungen die verabreichte Dosis (in µMol/kg p.o.) sowie die jeweils ermittelte Cholesterinresorption (CHORES) in Prozenten der Cholesterinresorption von unbehandelten Kontrolltieren. Ausserdem enthält die Tabelle Angaben über die akute Toxizität der untersuchten Verbindungen ($DL_{50}$ in mg/kg bei einmaliger oraler Verabreichung an Mäusen).

<u>Tabelle</u>

| Verbindung der Formel I | | | | | Dosis in µMol/kg p.o. | CHORES in % | $DL_{50}$ in mg/kg p.o. |
|---|---|---|---|---|---|---|---|
| $R^1$ | $R^2$ | $R^3$ | $R^4$ | $R^5$ bzw. abgewandeltes $R^5$ | | | |
| $CH_3$ | $CH_3$ | Cl | Br | $-NHC(CH_3)(CH_2OH)_2$ | 100 | 31 | > 5000 |
| $CH_3$ | $CH_3$ | Cl | Br | $-NHC(CH_2OH)_3$ | 100 | 30 | > 5000 |
| $CH_3$ | $CH_3$ | Cl | Br | $-NHCH_2CH(OH)CH_2OH$ | 100 | 24 | - |
| $CH_3$ | $CH_3$ | Cl | Br | 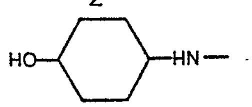 | 100 | 34 | - |
| $CH_3$ | $CH_3$ | Cl | Br | 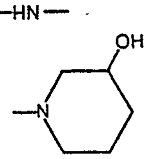 | 100 | 44 | - |
| $CH_3$ | $CH_3$ | Cl | Br | 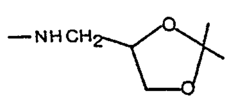 | 100 | 25 | - |

Die erfindungsgemässen Produkte können als Heilmittel, z.B. in Form pharmazeutischer Präparate, Verwendung finden. Die pharmazeutischen Präparate werden oral, z.B. in Form von Tabletten, Lacktabletten, Dragées, Hart- und Weichgelatinekapseln, Lösungen, Emulsionen oder Suspensionen, verabreicht.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend ein Benzodiazepin der Formel I, einen leicht hydrolysierbaren Ester und/oder Aether oder ein pharmazeutisch annehmbares Salz einer solchen Verbindung, ebenfalls Gegenstand der vorliegenden Erfindung, weiterhin auch ein Verfahren zur Herstellung solcher Arzneimittel, welches dadurch gekennzeichnet ist, dass man eine oder mehrere erfindungsgemässe Produkte und gegebenenfalls einen oder mehrere andere therapeutisch wertvolle Stoffe in eine galenische Darreichungsform bringt. Wie eingangs erwähnt können die erfindungsgemässen Produkte bei der Bekämpfung bzw. Verhütung von Krankheiten verwendet werden.

Sie können insbesondere bei der Bekämpfung bzw. Verhütung der Atherosklerose verwendet werden. Die Dosierung

kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 50 mg bis etwa 3 g, vorzugsweise von etwa 200 mg bis etwa 1 g angemessen sein.

Die nachfolgenden Beispiele sollen die vorliegende Erfindung näher erläutern, ihren Umfang jedoch in keiner Weise beschränken. Alle Temperaturen sind in Celsiusgraden angegeben.

## Beispiel 1

a) Man löst 53,6 g Carbobenzoxy-L-alanin in 400 ml trockenem Tetrahydrofuran, versetzt unter Eiskühlung tropfenweise mit 30 g Thionylchlorid und rührt während einer Stunde in der Kälte. Anschliessend wird eine Lösung von 54 g (0,2 Mol) 2-Amino-5-nitro-2'-chlorbenzophenon in 150 ml trockenem Tetrahydrofuran schnell dazugetropft, worauf man die Reaktionsmischung während 24 Stunden bei Raumtemperatur rührt. Man engt die erhaltene Lösung ein, versetzt den Rückstand mit Eis und 10-proz. Natriumbicarbonatlösung und extrahiert mit Methylenchlorid. Die organische Lösung wird über Natriumsulfat getrocknet und eingedampft. Den Rückstand versetzt man mit trockenem Aether, lässt während einer Stunde kristallisieren und filtriert dann unter Nachwaschen mit Aether/Petroläther (1:1). Nach dem Trocknen erhält man (S)-Benzyl-[1-[[2-(o-chlorbenzoyl)-4-nitrophenyl]carbamoyl]äthyl]carbamat vom Smp. 143-145°; $[\alpha]^{D}_{25}$ = -18°(1-proz. Lösung in Methylenchlorid).

b) Man löst 130 g (0,27 Mol) (S)-Benzyl-[1-[[2-(o-chlorbenzoyl)-4-nitrophenyl]carbamoyl]äthyl]carbamat in 350 ml einer 30 bis 33-proz. Lösung von Bromwasserstoff in Eisessig, rührt während 30 Minuten bei Raumtemperatur, dampft ein, versetzt mit Wasser und extrahiert dreimal mit Aether. Die wässerige Lösung wird in Eis gekühlt, mit festem Natriumbicarbonat neutralisiert und mit Methylenchlorid extrahiert. Man trocknet die organische Phase über Natriumsulfat und dampft ein. Den Rückstand versetzt man mit 80 ml Eisessig und 800 ml Toluol, erhitzt während 20 Minuten unter Rückfluss zum Sieden und dampft dann ein. Man nimmt das zurückbleibende Oel in Methylenchlorid auf, wäscht mit gesättigter Natriumbicarbonatlösung, trocknet über Natriumsulfat und dampft ein. Der Rückstand wird in 500 ml Benzol gelöst, worauf man mit racemischem 5-(o-chlorphenyl)-1,3-dihydro-3-methyl-7-nitro-2H-1,4-benzodiazepin-2-on animpft und über Nacht bei Raumtemperatur stehen lässt. Nach

Abfiltrieren vom kristallisierten Material wird die Mutterlauge eingedampft. Nach Umkristallisieren des Rückstandes aus Aether erhält man (S)-5-(o-Chlorphenyl)-1,3-dihydro-3-methyl-7-nitro-2H-1,4-benzodiazepin-2-on vom Smp. 200-202°; $[\alpha]^{D}_{25}$ = + 241° (1-proz. Lösung in Methylenchlorid).

c) 100 g (0,3 Mol) (S)-5-(o-Chlorphenyl)-1,3-dihydro-3-methyl-7-nitro-2H-1,4-benzodiazepin-2-on werden in 700 ml trockenem Aceton gelöst, worauf man mit 79 g gemahlenem Kaliumcarbonat und mit 31,4 ml Methyljodid versetzt und während 4 Stunden bei Raumtemperatur rührt. Man versetzt das Reaktionsgemisch mit 30 ml Eisessig, dampft ein, versetzt den Rückstand mit einem Eis/Wasser-Gemisch und extrahiert mehrmals mit Methylenchlorid. Die organische Phase wird mit Wasser gewaschen, über Natriumsulfat getrocknet, eingeengt und mit Methanol versetzt. Nach Abfiltrieren der ausgefallenen Kristalle erhält man (S)-5-(o-Chlorphenyl)-1,3-dihydro-1,3-dimethyl-7-nitro-2H-1,4-benzodiazepin-2-on vom Smp. 159°; $[\alpha]^{D}_{25}$ = + 381,2° (1-proz. Lösung in Methylenchlorid).

d) Man versetzt eine Lösung von 68,2 g (0,198 Mol) (S)-5-(o-Chlorphenyl)-1,3-dihydro-1,3-dimethyl-7-nitro-2H-1,4-benzodiazepin-2-on in einem Gemisch aus 400 ml Tetrahydrofuran und 950 ml Methanol mit Raney-Nickel, das man vorher mit Methanol und Tetrahydrofuran mehrmals gewaschen hat, und hydriert die Suspension unter Rühren und Wasserkühlung, worauf man filtriert und eindampft. Der Rückstand wird aus Aether umkristallisiert; man erhält (S)-7-Amino-5-(o-chlorphenyl)-1,3-dihydro-1,3-dimethyl-2H-1,4-benzodiazepin-2-on vom Smp. 221°; $[\alpha]^{D}_{25}$ = -13,8° (1-proz. Lösung in Methylenchlorid).

e) Man kühlt eine Lösung von 75 g (0,239 Mol) (S)-7-Amino-5-(o-chlorphenyl)-1,3-dihydro-1,3-dimethyl-2H-1,4-benzodiazepin-2-on in 1500 ml Eisessig auf 0°, versetzt rasch mit einer Lösung von 12,5 ml Brom in 350 ml Eisessig und rührt dann noch während 30 Minuten bei 0°. Das

Gemisch wird mit Eiswasser versetzt und mit Methylenchlorid extrahiert. Der organische Auszug wird über Natriumsulfat getrocknet und eingedampft. Man kristallisiert den erhaltenen Rückstand aus Methylenchlorid/Aether um und erhält (S)-7-Amino-6-brom-5-(o-chlorphenyl)-1,3-dihydro-1,3-dimethyl-2H-1,4-benzodiazepin-2-on vom Smp. 243°; $[\alpha]^{D}_{25}$ = 41,7° (1-proz. Lösung in Methylenchlorid).

f) Man löst 50 g (0,127 Mol) (S)-7-Amino-6-brom-5-(o-chlorphenyl)-1,3-dihydro-1,3-dimethyl-2H-1,4-benzodiazepin-2-on unter Erwärmen in 700 ml 1,2-Dichloräthan und tropft die noch heisse Lösung unter Rühren und Kühlen so zu einer Mischung aus 90 ml (0,173 Mol) einer 20-proz. Lösung von Phosgen in Toluol und 500 ml Dichloräthan, dass die Reaktionstemperatur 10° nicht übersteigt. Anschliessend erhitzt man das Reaktionsgemisch während 1 Stunde unter Rühren und unter Rückfluss zum Sieden, destilliert 500 ml 1,2-Dichloräthan ab, kühlt die Lösung auf 10° ab, stellt mit Triäthylamin basisch und dampft ein, wobei man (S)-[6-Brom-5-(o-chlorphenyl)-2,3-dihydro-1,3-dimethyl-2-oxo-1H-1,4-benzodiazepin-7-yl]isocyanat als Schaum erhält, der unter Feuchtigkeitsausschluss während mehreren Tagen im Eisschrank aufbewahrt werden kann.

g) Eine Lösung von (S)-[6-Brom-5-(o-chlorphenyl)-2,3-dihydro-1,3-dimethyl-2-oxo-1H-1,4-benzodiazepin-7-yl]isocyanat (erhalten gemäss Angaben in Beispiel 1f aus 5 g (0,0127 Mol) (S)-7-Amino-6-brom-5-(o-chlorphenyl)-1,3-dihydro-1,3-dimethyl-2H-1,4-benzodiazepin-2-on) in 1,2-Dichloräthan wird mit einer Dichloräthanlösung von 1,3 g 4-Hydroxypiperidin versetzt, während 1 Stunde bei Raumtemperatur gerührt und dann eingedampft. Den Rückstand nimmt man in Methylenchlorid auf, wäscht mit Wasser, trocknet über Natriumsulfat und dampft ein. Durch Chromatographieren an Kieselgel unter Eluieren mit Essigester und Umkristallisieren aus Aether erhält man N-[(S)-6-Brom-5-(o-chlorphenyl)-2,3-dihydro-1,3-dimethyl-2-oxo-1H-1,4-benzodiazepin-7-yl]-4-hydroxy-1-piperidin-carboxamid vom Smp. 140° (Zersetzung);

$[\alpha]^D_{25}$ = + 31,6° (1-proz. Lösung in Methylenchlorid).

## Beispiel 2

Eine Lösung von (S)-[6-Brom-5-(o-chlorphenyl)-2,3-dihydro-1,3-dimethyl-2-oxo-1H-1,4-benzodiazepin-7-yl]isocyanat (erhalten gemäss Angaben in Beispiel 1f aus 2,81 g (0,0072 Mol) (S)-7-Amino-6-brom-5-(o-chlorphenyl)-1,3-dihydro-1,3-dimethyl-2H-1,4-benzodiazepin-2-on) in Aceton wird mit einer Lösung von 1,3 g (0,0107 Mol) Tris(hydroxymethyl)amino-methan in 5 ml Wasser versetzt, worauf man während 15 Minuten bei Raumtemperatur rührt, mit Essigester extrahiert und den organischen Auszug über Natriumsulfat trocknet und eindampft. Der Rückstand wird aus Tetrahydrofuran/1,2-Dichloräthan umkristallisiert und liefert 1-[(S)-6-Brom-5-(o-chlorphenyl)-2,3-dihydro-1,3-dimethyl-2-oxo-1H-1,4-benzodiazepin-7-yl]-3-[2-hydroxy-1,1-bis(hydroxymethyl)äthyl]harnstoff vom Smp. 175° (Zersetzung); $[\alpha]^D_{25}$ = + 22,87°(0,8-proz. Lösung in Methanol).

## Beispiel 3

Eine Lösung von (S)-[6-Brom-5-(o-chlorphenyl)-2,3-dihydro-1,3-dimethyl-2-oxo-1H-1,4-benzodiazepin-7-yl]isocyanat (erhalten gemäss Angaben in Beispiel 1f aus 5 g (0,0127 Mol) (S)-7-Amino-6-brom-5-(o-chlorphenyl)-1,3-dihydro-1,3-dimethyl-2H-1,4-benzodiazepin-2-on) in Tetrahydrofuran wird mit einer Lösung von 5 g (0,039 Mol) 2-Amino-1,3-propandiol-hydrochlorid und 4 g (0,04 Mol) Kaliumbicarbonat in 20 ml Wasser versetzt. Das Gemisch wird während 30 Minuten bei Raumtemperatur gerührt, worauf man das Tetrahydrofuran abdampft und den Rückstand mehrmals mit Essigester extrahiert. Die organische Phase wird über Natriumsulfat getrocknet und eingedampft, worauf man den Rückstand aus Methylenchlorid umkristallisiert. Man erhält 1-[(S)-6-Brom-5-(o-chlorphenyl)-2,3-dihydro-1,3-dimethyl-2-oxo-1H-1,4-benzodiazepin-7-yl]-3-[2-hydroxy-1-(hydroxymethyl)äthyl]harnstoff vom Smp. 184° (Zersetzung); $[\alpha]^D_{25}$ =

+23,63° (0,8-proz. Lösung in Methanol).

Beispiel 4

Eine Lösung von (S)-[6-Brom-5-(o-chlorphenyl)-2,3-dihydro-1,3-dimethyl-2-oxo-1H-1,4-benzodiazepin-7-yl]isocyanat (erhalten gemäss Angaben in Beispiel 1f aus 5 g (0,0127 Mol) (S)-7-Amino-6-brom-5-(o-chlorphenyl)-1,3-dihydro-1,3-dimethyl-2H-1,4-benzodiazepin-2-on) in Tetrahydrofuran wird mit einer Lösung von 4,4 g (0,043 Mol) 3-Amino-1,2-propandiol in 50 ml Tetrahydrofuran und 10 ml Wasser versetzt. Das Gemisch wird während einer Stunde bei Raumtemperatur gerührt, und mit Methylenchlorid extrahiert, worauf man den organischen Auszug über Natriumsulfat trocknet und eindampft. Der Rückstand wird unter Eluieren mit Essigester/Methanol (95:5) an Kieselgel chromatographiert und dann aus Aether umkristallisiert. Man erhält 1-[(S)-6-Brom-5-(o-chlorphenyl)-2,3-dihydro-1,3-dimethyl-2-oxo-1H-1,4-benzodiazepin-7-yl]-3-(2,3-dihydroxypropyl)harnstoff vom Smp. 175°; $[\alpha]^{D}_{25}$ = + 57,5°(0,8-proz. Lösung in Dioxan).

Beispiel 5

Eine Lösung von (S)-[6-Brom-5-(o-chlorphenyl)-2,3-dihydro-1,3-dimethyl-2-oxo-1H-1,4-benzodiazepin-7-yl]isocyanat (erhalten gemäss Angaben in Beispiel 1f aus 2,8 g (0,0072 Mol) (S)-7-Amino-6-brom-5-(o-chlorphenyl)-1,3-dihydro-1,3-dimethyl-2H-1,4-benzodiazepin-2-on) in Aceton wird mit einer Lösung von 2,4 ml 4-Amino-cyclohexanol in 10 ml Aceton versetzt, während 15 Minuten bei Raumtemperatur gerührt, mit Wasser versetzt und mit Essigester extrahiert. Die Essigesterlösung wird über Natriumsulfat getrocknet und eingedampft, worauf man den Rückstand unter Eluieren mit Chloroform und Essigester an Kieselgel chromatographiert und aus Methylenchlorid umkristallisiert. Man erhält 1-[(S)-6-Brom-5-(o-chlorphenyl)-2,3-dihydro-1,3-dimethyl-2-oxo-1H-1,4-benzodiazepin-7-yl]-3-(4-hydroxycyclohexyl)-harnstoff vom Smp. 226-227°; $[\alpha]^{D}_{25}$ = + 22,63° (0,8-proz.

Lösung in Methanol).

## Beispiel 6

Eine Lösung von (S)-[6-Brom-5-(o-chlorphenyl)-2,3-dihydro-1,3-dimethyl-2-oxo-1H-1,4-benzodiazepin-7-yl]isocyanat (erhalten gemäss Angaben in Beispiel 1f aus 15 g (0,038 Mol) (S)-7-Amino-6-brom-5-(o-chlorphenyl-1,3-dihydro-1,3-dimethyl-2H-1,4-benzodiazepin-2-on) in Aceton wird mit 4,02 g (0,038 Mol) 2-Amino-2-methyl-1,3-propandiol versetzt, während einer Stunde bei Raumtemperatur gerührt und dann eingedampft. Den Rückstand chromatographiert man unter Eluieren mit Essigester an Kieselgel. Nach Umkristallisieren aus Aceton/Hexan erhält man 1-[(S)-6-Brom-5-(o-chlorphenyl)-2,3-dihydro-1,3-dimethyl-2-oxo-1H-1,4-benzodiazepin-7-yl]-3-[1,1-bis(hydroxymethyl)äthyl]harnstoff vom Smp. 190° (Zersetzung); $[\alpha]^{D}_{25}$ = + 25,2° (0,8-proz. Lösung in Methanol).

## Beispiel 7

In Analogie zu den Angaben in Beispiel 1g, jedoch unter Verwendung von 3-Hydroxy-piperidin anstelle von 4-Hydroxypiperidin, erhält man N-[(S)-6-Brom-5-(o-chlorphenyl)-2,3-dihydro-1,3-dimethyl-2-oxo-1H-1,4-benzodiazepin-7-yl]-3-hydroxy-1-piperidin-carboxamid vom Smp. 140° (Zersetzung); $[\alpha]^{D}_{25}$ = + 30,8° (1-proz. Lösung in Methylenchlorid).

## Beispiel 8

Eine Lösung von (S)-[6-Brom-5-(o-chlorphenyl-2,3-dihydro-1,3-dimethyl-2-oxo-1H-1,4-benzodiazepin-7-yl]isocyanat (erhalten gemäss Angaben in Beispiel 1f aus 10 g (0,0254 Mol) (S)-7-Amino-6-brom-5-(o-chlorphenyl)-1,3-dihydro-1,3-dimethyl-2H-1,4-benzodiazepin-2-on) in Aceton wird mit einer Lösung von 16 g D-Glucosamin-Hydrochlorid und 7,3 g Kaliumcarbonat in 100 ml Wasser versetzt, während

10 Minuten bei Raumtemperatur gerührt und mehrmals mit Essigester extrahiert. Die organische Phase wird über Natriumsulfat getrocknet und eingedampft, worauf man den Rückstand aus Tetrahydrofuran/Methylenchlorid umkristallisiert. Man erhält 2-[3-[(S)-6-Brom-5-(o-chlorphenyl)-2,3-dihydro-1,3-dimethyl-2-oxo-1H-1,4-benzodiazepin-7-yl]ureido]-2-deoxy-D-glucose als Diastereomerengemisch mit einem Smp. von 185° (Zersetzung); $[\alpha]^{D}_{25} = + 45°$ (0,8-proz. Lösung in Methanol).

## Beispiel 9

Eine Lösung von (S)-[6-Brom-5-(o-chlorphenyl)-2,3-dihydro-1,3-dimethyl-2-oxo-1H-1,4-benzodiazepin-7-yl]isocyanat (erhalten gemäss Angaben in Beispiel 1f aus 4 g (0,01 Mol) (S)-7-Amino-6-brom-5-(o-chlorphenyl)-1,3-dihydro-1,3-dimethyl-2H-1,4-benzodiazepin-2-on) in Aceton wird mit einer Lösung von 3,8 g (0,04 Mol) 3-Amino-1,2-propandiol in 10 ml Wasser und 50 ml Aceton versetzt, während 12 Stunden bei Raumtemperatur gerührt und mit Methylenchlorid extrahiert. Der organische Auszug wird über Natriumsulfat getrocknet und eingedampft, worauf man den Rückstand unter Eluieren mit Chloroform an Kieselgel chromatographiert. Man erhält 1-[(S)-6-Brom-5-(o-chlorphenyl)-2,3-dihydro-1,3-dimethyl-2-oxo-1H-1,4-benzodiazepin-7-yl]-3-[(2,2-dimethyl-1,3-dioxolan-4-yl)methyl]harnstoff vom Smp. 135° (Zersetzung); $[\alpha]^{D}_{25} = + 7,6°$ (0,8-proz. Lösung in Methanol).

## Beispiel 10

Eine Lösung von 5 g 1-[(S)-6-Brom-5-(o-chlorphenyl)-2,3-dihydro-1,3-dimethyl-2-oxo-1H-1,4-benzodiazepin-7-yl]-3-[1,1-bis(hydroxymethyl)äthyl]harnstoff in Pyridin wird mit 4 ml Acetanhydrid versetzt, während 1 Stunde bei Raumtemperatur gerührt, mit Wasser versetzt und mit Methylenchlorid extrahiert. Die organische Phase wird über Natriumsulfat getrocknet und eingedampft, worauf man den Rückstand

unter Eluieren mit Chloroform an Kieselgel chromatographiert und dann aus Petroläther umkristallisiert. Man erhält (S)-N-[2-(Acetyloxy)-1-[(acetyloxy)methyl]-1-methyläthyl]-N'-[6-brom-5-(o-chlorphenyl)-2,3-dihydro-1,3-dimethyl-2-oxo-1H-1,4-benzodiazepin-7-yl]harnstoff vom Smp. 105-107° (Zersetzung); $[\alpha]^{D}_{25}$ = + 33,75° (0,8-proz. Lösung in Methylenchlorid).

## Beispiel 11

a) Eine Lösung von (S)-[6-Brom-5-(o-chlorphenyl)-2,3-dihydro-1,3-dimethyl-2-oxo-1H-1,4-benzodiazepin-7-yl]-isocyanat (erhalten gemäss Angaben in Beispiel 1f aus 4 g (0,01 Mol) (S)-7-Amino-6-brom-5-(o-chlorphenyl)-1,3-dihydro-1,3-dimethyl-2H-1,4-benzodiazepin-2-on) in Aceton wird mit einer Lösung von 1,44 g (0,011 Mol) 2,2-Dimethyl-1,3-dioxolan-4-methylamin in Aceton versetzt und während 12 Stunden bei Raumtemperatur gerührt. Das Aceton wird abgedampft, worauf man den Rückstand unter Eluieren mit Chloroform an Kieselgel chromatographiert. Man erhält 1-[(S)-6-Brom-5-(o-chlorphenyl)-2,3-dihydro-1,3-dimethyl-2-oxo-1H-1,4-benzodiazepin-7-yl]-3-[(2,2-dimethyl-1,3-dioxolan-4-yl)methyl]harnstoff vom Schmelzpunkt 135° (Zersetzung).

b) Eine Lösung von 1-[(S)-6-Brom-5-(o-chlorphenyl)-2,3-dihydro-1,3-dimethyl-2-oxo-1H-1,4-benzodiazepin-7-yl]-3-[(2,2-dimethyl-1,3-dioxolan-4-yl)methyl]harnstoff in Aethanol wird mit 1N-Salzsäure während 5 Stunden bei Raumtemperatur gerührt.Man extrahiert mit Essigester, wäscht mit Wasser, trocknet über Natriumsulfat und dampft ein. Der Rückstand wird unter Eluieren mit Essigester/Methanol (95:5) an Kieselgel chromatographiert. Nach Umkristallisieren aus Methanol/Aether erhält man 1-[(S)-6-Brom-5-(o-chlorphenyl)-2,3-dihydro-1,3-dimethyl-2-oxo-1H-1,4-benzodiazepin-7-yl]-3-(2,3-dihydroxypropyl)harnstoff vom Schmelzpunkt 175° (Zersetzung).

Beispiel A

1-[(S)-6-Brom-5-(o-chlorphenyl)-2,3-dihydro-1,3-dimethyl-2-oxo-1H-1,4-benzodiazepin-7-yl]-3-[1,1-bis(hydroxymethyl)äthyl]harnstoff kann wie folgt als Wirkstoff zur Herstellung von pharmazeutischen Präparaten verwendet werden:

a) Tabletten

| Tabletten | 1 Tablette enthält |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Der Wirkstoff wird mit der Hälfte der mikrokristallinen Cellulose gemischt und mit einer 10-proz. Lösung von Hydroxypropylmethylcellulose in einem Gemisch aus Isopropanol und Methylenchlorid granuliert. Das Granulat wird getrocknet, gesiebt und mit dem Rest der Hilfsstoffe gemischt. Alsdann wird es auf einer Presse zu biplanen Tabletten von 12 mm Durchmesser mit Kreuzbruchrille verpresst.

b) Kapseln

| Kapseln | 1 Kapsel enthält |
|---|---|
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

Der Wirkstoff wird mit den Hilfsstoffen gemischt und gesiebt. Nach erneutem Mischen wird die erhaltene Kapselfüllmasse auf einer vollautomatischen Kapselabfüllmaschine in Gelatinesteckkapseln geeigneter Grösse abgefüllt.

## Patentansprüche

1. Benzodiazepine der allgemeinen Formel

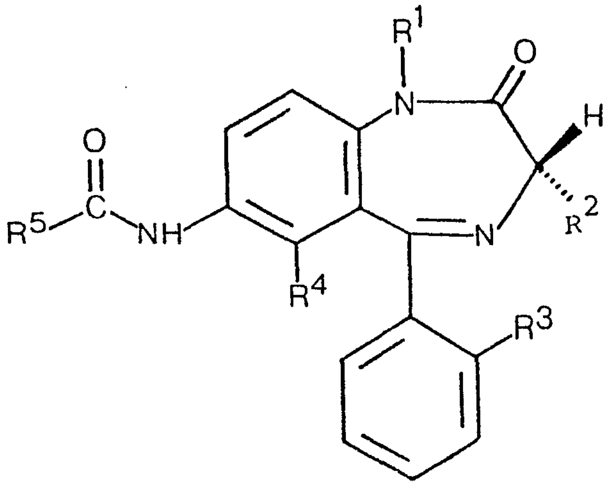

I

worin $R^1$ $(C_1-C_4)$-Alkyl, $R^2$ Wasserstoff oder Methyl, $R^3$ und $R^4$ je Halogen und $R^5$ durch mindestens zwei Hydroxygruppen substituiertes $(C_3-C_9)$-Alkylamino, durch mindestens eine Hydroxygruppe substituiertes $(C_3-C_6)$-Cycloalkylamino, Glucosamino, Galactosamino, Mannosamino, Monohydroxy-1-azetidinyl, Mono- oder Dihydroxy-1-pyrrolidinyl oder Mono-, Di- oder Trihydroxy-1-piperidinyl, bedeuten,

leicht hydrolysierbare Ester und Aether davon, sowie pharmazeutisch annehmbare Salze von diesen Verbindungen.

2. Produkte gemäss Anspruch 1, dadurch gekennzeichnet, dass die leicht hydrolysierbaren Ester Ester bzw. Halbester von niederen Alkancarbonsäuren oder niederen Alkandicarbonsäuren, cyclische Diester von niederen Alkandicarbonsäuren oder Ester von aromatischen oder araliphatischen Carbonsäuren sind, und dass die leicht hydrolysierbaren Aether offenkettige oder cyclische Acetale oder Ketale oder niedere Alkyläther sind.

3. Benzodiazepine der in Anspruch 1 definierten Formel I und pharmazeutisch annehmbare Säureadditionssalze davon.

4. Produkte gemäss einem der Ansprüche 1 bis 3, worin $R^1$ Methyl bedeutet.

5. Produkte gemäss einem der Ansprüche 1 bis 4, worin $R^2$ Methyl bedeutet.

6. Produkte gemäss einem der Ansprüche 1 bis 5, worin $R^3$ Chlor bedeutet.

7. Produkte gemäss einem der Ansprüche 1 bis 6, worin $R^4$ Brom bedeutet.

8. Verbindungen gemäss einem der Ansprüche 3 bis 7, worin $R^5$ durch mindestens zwei Hydroxygruppen substituiertes $(C_3-C_9)$-Alkylamino bedeutet.

9. 1-[(S)-6-Brom-5-(o-chlorphenyl)-2,3-dihydro-1,3-dimethyl-2-oxo-1H-1,4-benzodiazepin-7-yl]-3-[1,1-bis-(hydroxymethyl)äthyl]harnstoff.

10. 1-[(S)-6-Brom-5-(o-chlorphenyl)-2,3-dihydro-1,3-dimethyl-2-oxo-1H-1,4-benzodiazepin-7-yl]-3-[(2,2-dimethyl-1,3-dioxolan-4-yl)methyl]harnstoff und (S)-N-[2-(Acetyloxy)-1-[(acetyloxy)methyl]-1-methyläthyl]-N'-[6-brom-5-(o-chlorphenyl)-2,3-dihydro-1,3-dimethyl-2-oxo-1H-1,4-benzodiazepin-7-yl]harnstoff.

11. 1-[(S)-6-Brom-5-(o-chlorphenyl)-2,3-dihydro-1,3-dimethyl-2-oxo-1H-1,4-benzodiazepin-7-yl]-3-[2-hydroxy-1,1-bis(hydroxymethyl)äthyl]harnstoff, 1-[(S)-6-Brom-5-(o-chlorphenyl)-2,3-dihydro-1,3-dimethyl-2-oxo-1H-1,4-benzodiazepin-7-yl]-3-[2-hydroxy-1-(hydroxymethyl)äthyl]-harnstoff, 1-[(S)-6-Brom-5-(o-chlorphenyl)-2,3-dihydro-1,3-dimethyl-2-oxo-1H-1,4-benzodiazepin-7-yl]-3-(2,3-dihydroxypropyl)harnstoff, 1-[(S)-6-Brom-5-(o-chlorphenyl)-2,3-dihydro-1,3-dimethyl-2-oxo-1H-1,4-benzodiazepin-7-yl]-3-(4-hydroxycyclohexyl)harnstoff, N-[(S)-6-Brom-5-(o-chlorphenyl)-2,3-dihydro-1,3-dimethyl-2-oxo-1H-1,4-benzodiazepin-7-yl]-4-hydroxy-1-piperidincarboxamid, N-[(S)-6-Brom-5-(o-chlorphenyl)-2,3-dihydro-1,3-dimethyl-2-oxo-1H-1,4-benzodiazepin-7-yl]-3-hydroxy-1-piperidincarboxamid und 2-[3-[(S)-6-Brom-5-(o-chlorphenyl)-2,3-dihydro-1,3-dimethyl-2-oxo-1H-1,4-benzodiazepin-7-yl]ureido]-2-deoxy-D-glucose.

12. Produkte gemäss einem der Ansprüche 1 bis 11 als pharmazeutische Wirkstoffe.

13. Produkte gemäss einem der Ansprüche 1 bis 11 als die intestinale Resorption von Cholesterin hemmende Wirkstoffe.

14. 1-[(S)-6-Brom-5-(o-chlorphenyl)-2,3-dihydro-1,3-dimethyl-2-oxo-1H-1,4-benzodiazepin-7-yl]-3-[1,1-bis(hydroxymethyl)äthyl]harnstoff als pharmazeutischer Wirkstoff.

15. 1-[(S)-6-Brom-5-(o-chlorphenyl)-2,3-dihydro-1,3-dimethyl-2-oxo-1H-1,4-benzodiazepin-7-yl]-3-[1,1-bis-(hydroxymethyl)äthyl]harnstoff als die intestinale Resorption von Cholesterin hemmender Wirkstoff.

16. Verfahren zur Herstellung von Produkten gemäss einem der Ansprüche 1 bis 11, dadurch gekennzeichnet, dass man

a) ein Benzodiazepin der allgemeinen Formel

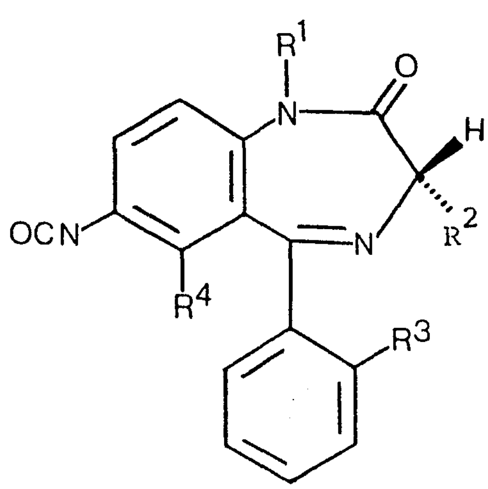

II

worin $R^1$, $R^2$, $R^3$ und $R^4$ die in Anspruch 1 angegebene Bedeutung besitzen,

mit einer Verbindung der allgemeinen Formel

$$R^5\text{-}H \qquad \text{III}$$

worin $R^5$ die in Anspruch 1 angegebene Bedeutung besitzt,

oder mit einem Derivat einer Verbindung der Formel III, worin die Hydroxygruppe(n) allesamt oder teilweise in Form leicht hydrolysierbarer Ester und/oder Aether vorliegt bzw. vorliegen, umsetzt,

b) erwünschtenfalls einen erhaltenen leicht hydrolysierbaren Ester oder Aether eines Benzodiazepins der Formel I hydrolysiert, und/oder

c) erwünschtenfalls in einer erhaltenen Verbindung die freie(n) Hydroxygruppe(n) allesamt oder teilweise in leicht hydrolysierbare Ester und/oder Aether überführt und/oder

d) erwünschtenfalls ein erhaltenes Benzodiazepin der Formel I oder einen leicht hydrolysierbaren Ester oder Aether davon in ein pharmazeutisch annehmbares Salz überführt.

17. Arzneimittel, enthaltend ein Produkt gemäss einem der Ansprüche 1 bis 11.

18. Die intestinale Resorption von Cholesterin hemmende Mittel, enthaltend ein Produkt gemäss einem der Ansprüche 1 bis 11.

19. Arzneimittel, enthaltend 1-[(S)-6-Brom-5-(o-chlorphenyl)-2,3-dihydro-1,3-dimethyl-2-oxo-1H-1,4-benzodiazepin-7-yl]-3-[1,1-bis(hydroxymethyl)äthyl]harnstoff.

20. Die intestinale Resorption von Cholesterin hemmende Mittel, enthaltend 1-[(S)-6-Brom-5-(o-chlorphenyl)-2,3-dihydro-1,3-dimethyl-2-oxo-1H-1,4-benzodiazepin-7-yl]-3-[1,1-bis(hydroxymethyl)äthyl]harnstoff.

***

Patentansprüche
OESTERREICH.

1. Verfahren zur Herstellung von Benzodiazepinen der allgemeinen Formel

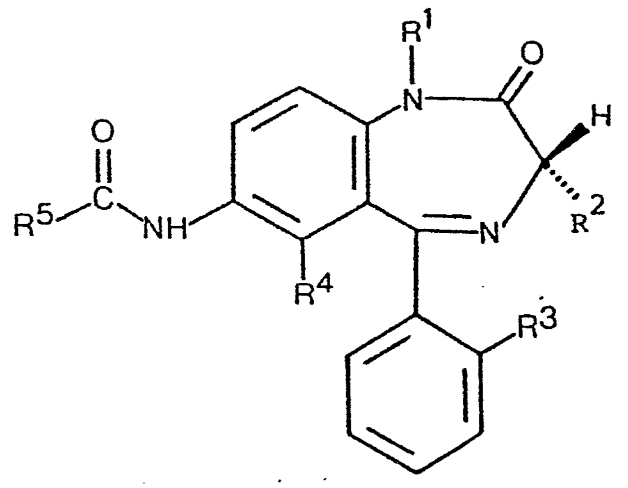

I

worin $R^1$ $(C_1-C_4)$-Alkyl, $R^2$ Wasserstoff oder Methyl, $R^3$ und $R^4$ je Halogen und $R^5$ durch mindestens zwei Hydroxygruppen substituiertes $(C_3-C_9)$-Alkylamino, durch mindestens eine Hydroxygruppe substituiertes $(C_3-C_6)$-Cycloalkylamino, Glucosamino, Galactosamino, Mannosamino, Monohydroxy-1-azetidinyl, Mono- oder Dihydroxy-1-pyrrolidinyl oder Mono-, Di- oder Trihydroxy-1-piperidinyl bedeuten,

von leicht hydrolysierbaren Estern und Aethern davon, sowie von pharmazeutisch annehmbaren Salzen von diesen Verbindungen, dadurch gekennzeichnet, dass man

a) ein Benzodiazepin der allgemeinen Formel

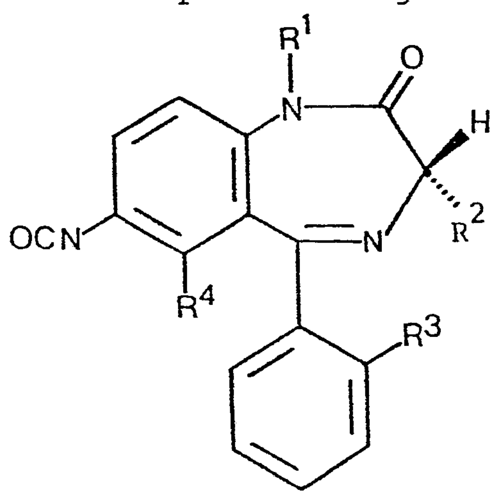

II

worin $R^1$, $R^2$, $R^3$ und $R^4$ obige Bedeutung besitzen, mit einer Verbindung der allgemeinen Formel

$$R^5\text{-}H \qquad III$$

worin $R^5$ obige Bedeutung besitzt, oder mit einem Derivat einer Verbindung der Formel III, worin die Hydroxygruppe(n) allesamt oder teilweise in Form leicht hydrolysierbarer Ester und/oder Aether vorliegt bzw. vorliegen, umsetzt,

b) erwünschtenfalls einen erhaltenen leicht hydrolysierbaren Ester oder Aether eines Benzodiazepins der Formel I hydrolysiert, und/oder

c) erwünschtenfalls in einer erhaltenen Verbindung die freien Hydroxygruppen allesamt oder teilweise in leicht hydrolysierbare Ester und/oder Aether überführt und/oder

d) erwünschtenfalls ein erhaltenes Benzodiazepin der Formel I oder einen leicht hydrolysierbaren Ester oder Aether davon in ein pharmazeutisch annehmbares Salz überführt.

2. Verfahren gemäss Anspruch 1, dadurch gekennzeichnet, dass die leicht hydrolysierbaren Ester Ester bzw. Halbester von niederen Alkancarbonsäuren oder niederen Alkandicarbonsäuren, cyclische Diester von niederen Alkandicarbonsäuren oder Ester von aromatischen oder araliphatischen Carbonsäuren sind, und dass die leicht hydrolysierbaren Aether offenkettige oder cyclische Acetale oder Ketale oder niedere Alkyläther sind.

3. Verfahren gemäss Anspruch 1, dadurch gekennzeichnet, dass man Benzodiazepine der in Anspruch 1 definierten Formel I und pharmazeutisch annehmbare Säureadditionssalze davon herstellt.

4. Verfahren gemäss einem der Ansprüche 1 bis 3, dadurch gekennzeichnet, dass $R^1$ Methyl bedeutet.

5. Verfahren gemäss einem der Ansprüche 1 bis 4, dadurch gekennzeichnet, dass $R^2$ Methyl bedeutet.

6. Verfahren gemäss einem der Ansprüche 1 bis 5, dadurch gekennzeichnet, dass $R^3$ Chlor bedeutet.

7. Verfahren gemäss einem der Ansprüche 1 bis 6, dadurch gekennzeichnet, dass $R^4$ Brom bedeutet.

8. Verfahren gemäss einem der Ansprüche 3 bis 7, dadurch gekennzeichnet, dass $R^5$ durch mindestens zwei Hydroxygruppen substituiertes $(C_3-C_9)$-Alkylamino bedeutet.

9. Verfahren gemäss Anspruch 1, dadurch gekennzeichnet, dass man 1-[(S)-6-Brom-5-(o-chlorphenyl)-2,3-dihydro-1,3-dimethyl-2-oxo-1H-1,4-benzodiazepin-7-yl]-3-[1,1-bis(hydroxymethyl)äthyl]harnstoff herstellt.

Europäisches Patentamt

# EUROPÄISCHER RECHERCHENBERICHT

0084357

Nummer der Anmeldung

EP 83 10 0295

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | US-A-4 251 443 (A.E. FISCHLI)<br>* Insgesamt *<br>--- | 1-20 | C 07 D 243/26<br>C 07 D 401/12<br>C 07 D 405/12<br>C 07 H 13/12<br>A 61 K 31/55 //<br>C 07 C 125/065 |
| X | EP-A-0 008 421 (HOFFMANN-LA ROCHE)<br>* Insgesamt *<br>----- | 1-20 | |
| | | | RECHERCHIERTE SACHGEBIETE (Int. Cl. 3) |
| | | | C 07 D 243/00<br>C 07 D 401/00<br>C 07 D 405/00<br>C 07 H 13/00 |

Der vorliegende Recherchenbericht wurde für alle Patentansprüche erstellt.

| Recherchenort | Abschlußdatum der Recherche | Prüfer |
|---|---|---|
| DEN HAAG | 18-04-1983 | ALLARD M.S. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82